# FASCICULE DE BREVET EUROPEEN

(11) **EP 3 322 474 B1**
(45) Date de publication et mention de la délivrance du brevet: **29.01.2020**
(21) Numéro de dépôt: 16747544.1
(22) Date de dépôt: 15.07.2016
(51) Int. Cl.: A61N 1/05, A61B 5/0488, A61B 5/20, A61N 1/36, A61B 5/22, A63B 23/20

(54) **DISPOSITIF DE SONDE POUR LA GESTION DE L'INCONTINENCE URINAIRE A L'EFFORT**
SONDENVORRICHTUNG ZUR VERWALTUNG VON STRESSBEDINGTER HARNINKONTINENZ
PROBE DEVICE FOR MANAGING STRESS URINARY INCONTINENCE

(30) Priorité: 15.07.2015 FR 1556685; 06.04.2016 FR 1653023
(43) Date de publication de la demande: 23.05.2018
(73) Titulaire: Akse, 77550 Moissy-Cramayel (FR)
(72) Inventeur: BILLARD, Georges, 34470 Perols (FR)
(74) Mandataire: Rhein, Alain
(86) Numéro de dépôt international: PCT/FR2016/051824
(87) Numéro de publication internationale: WO 2017/009584

(56) Documents cités:
- EP-A1- 0 638 329
- FR-A1- 2 827 520
- FR-A1- 2 930 713
- US-A- 4 515 167
- US-A1- 2003 083 590
- US-A1- 2009 222 058
- OHLSSON B L ET AL: "MINIATURISED DEVICE FOR LONG-TERM INTRAVAGINAL ELECTRICAL STIMULATION FOR THE TREATMENT OF URINARY INCONTINENCE", MEDICAL AND BIOLOGICAL ENGINEERING AND COMPUTING, SPRINGER, HEILDELBERG, DE, vol. 26, no. 5, septembre 1988 (1988-09), pages 509-515, XP000004444, ISSN: 0140-0118, DOI: 10.1007/BF02441919

## Description

La présente invention concerne le domaine de la gestion de l'incontinence à l'effort, et en particulier, mais non limitativement, des incontinences urinaires.

L'invention concerne plus particulièrement un dispositif innovant permettant d'éviter, de manière permanente, notamment l'incontinence urinaire à l'effort, également appelée incontinence urinaire d'effort.

Il existe différents types d'incontinence urinaire, tous caractérisés par une perte involontaire des urines via le canal de sortie de la vessie, l'urètre.

Four ce qui est de l'incontinence urinaire à l'effort, celle-ci se caractérise plus particulièrement par une perte involontaire d'urine via l'urètre suite à un effort physique, en particulier une quinte de toux, des éternuements, des rires ou encore un soulèvement d'une charge plus ou moins lourde.

Cet effort physique, quel qu'il soit, va engendrer une augmentation de la pression au niveau de l'enceinte abdominale. La pression abdominale est ensuite naturellement transférée sur la vessie. Lorsque les muscles du périnée et du sphincter urinaire ne fonctionnent pas suffisamment pour contrer cette augmentation de pression et maintenir la vessie fermée, un écoulement d'urine, peu abondant et en jet, est susceptible de se produire, et cela sans sensation de besoin ressentie au préalable.

Au-delà de la gêne, voire de la honte, ressentie par la personne affectée d'incontinence urinaire, ce trouble peut engendrer des répercussions plus graves, notamment au niveau psychologique ou hygiénique. En outre, l'incontinence urinaire peut entrainer des irritations de la peau.

Traditionnellement, le diagnostic d'une incontinence urinaire est posé suite à un simple interrogatoire de la personne. Par ce biais, le spécialiste va notamment tenter de rechercher les antécédents, en particulier obstétricaux et de chirurgie pelvienne.

Il est également proposé, depuis plusieurs décennies, de diagnostiquer un problème d'incontinence en procédant à une exploration urodynamique au cours de laquelle on va mesurer l'évolution des pressions dans la vessie et dans le rectum, suite à l'application de certains stimuli mimant les conditions dans lesquelles se produisent les fuites urinaires.

Cette exploration urodynamique est effectuée au moyen d'une sonde introduite dans la vessie via l'urètre, ladite sonde étant reliée, par l'intermédiaire d'un lien filaire, à des moyens de stimulation (remplissage vésical) et d'enregistrement d'une réponse, ces moyens étant particulièrement imposants et positionnés à l'extérieur du corps du patient.

Une fois le diagnostic d'incontinence urinaire à l'effort posé, il existe, dans l'état de la technique, différentes solutions qui sont proposées aux personnes atteintes de ce désagrément.

Pour les femmes souffrant d'incontinence d'effort, il est souvent proposé, en première intention, des séances de rééducation périnéale visant notamment à remuscler le pelvis et à contrôler plus efficacement le fonctionnement de la musculature du périnée, dans le but d'améliorer également le contrôle de la vessie et la pression de fermeture de l'urètre.

Les méthodes existantes sont multiples, et on applique notamment le biofeedback et l'électrostimulation qui facilitent l'exécution des exercices des muscles du plancher pelvien chez les hommes et chez les femmes.

Toutefois, pour être efficaces, les séances de rééducation périnéale doivent être effectuées de manière régulière chez un praticien expérimenté, par exemple un kinésithérapeute, ce qui peut s'avérer particulièrement contraignant, En outre, les résultats obtenus ne sont pas toujours satisfaisants.

De ce fait, de nombreux patients atteints de fuites urinaires sont contraints de porter des protections spécifiques, adaptables en fonction des besoins des patients et de l'importance des fuites, telles que des lingettes, des couches ou encore des culottes jetables ou réutilisables.

Cependant, ces dispositifs ne permettent pas d'éviter les fuites et, outre l'inconfort qu'ils procurent, le port de ces protections peut être ressenti comme particulièrement gênant ou dégradant.

Pour remédier à cela, il a été imaginé d'implanter des bandelettes en tissu synthétique sous l'urètre dans le but de le soutenir, lesdites bandelettes étant rattachées aux tissus ou structures de la paroi abdominale ou de l'espace rétropubien.

Cependant, cette solution présente l'inconvénient de nécessiter une intervention chirurgicale et, de ce fait, une hospitalisation. En outre, à moyen ou à long terme, les bandelettes perdraient de leur efficacité, entraînant un retour de l'incontinence urinaire ou, plus grave encore, conduisant à une rétention par fibrose de l'urètre.

Par conséquent, les personnes souffrant d'incontinence sont finalement contraintes à revenir aux dispositifs de protection contre les fuites évoquées précédemment.

Aussi, pour récapituler, une fois que le diagnostic d'incontinence urinaire d'effort a été établi par un spécialiste, les solutions qui sont proposées actuellement ne permettent pas de remédier, de manière simple, satisfaisante et durable, aux problèmes des fuites urinaires.

Des sondes endocavitaires pouvant être introduites dans la cavité vaginale ou rectale sont déjà connues de l'art antérieur, par exemple dans les documents US 2009/0222058, EP 2 930 713, US 2003/0083590, US 4 515 167 et EP 2 827 520.

L'invention offre la possibilité de pallier les divers inconvénients de l'état de la technique en proposant un dispositif autonome et endocavitaire, destiné à être inséré par exemple dans le vagin ou dans le rectum et permettant d'envoyer une stimulation appropriée, au moment opportun, afin d'engendrer une contraction des muscles périnéaux, évitant par conséquent une perte de liquide urinaire.

A cet effet, la présente invention concerne un dispositif de sonde autonome et endocavitaire pour la gestion de l'incontinence à l'effort, ladite sonde étant apte à être introduite dans la cavité vaginale ou dans la cavité rectale d'une personne, le dispositif de sonde comportant au moins un moyen d'alimentation autonome en énergie électrique qui alimente, d'une part, un moyen de mesure de la variation d'un paramètre résultant d'un effort physique, et d'autre part, au moins un moyen de stimulation musculaire électrique au contact d'au moins un muscle apte à maintenir la continence urinaire, ledit moyen de stimulation étant déclenché en cas de variation dudit paramètre.

Le dispositif de sonde de l'invention se caractérise par le fait qu'il est configuré, d'une part, pour mesurer, à l'aide des moyens de mesure une augmentation de la pression au sein de la cavité vaginale ou rectale et, d'autre part, pour activer le moyen de stimulation musculaire électrique de sorte que l'intensité et la pente d'établissement de la stimulation sont proportionnelles à la vitesse d'établissement et au niveau de pression mesurée par le moyen de mesure. Selon une première caractéristique de l'invention, le dispositif de sonde est configuré pour activer la stimulation à un seuil inférieur au seuil de pression à partir duquel se produit la fuite.

Selon une deuxième caractéristique de l'invention, le paramètre mesuré consiste en un courant électrique accompagnant l'activité musculaire, faible mais existante, notamment du périnée, cette activité résultant d'un effort physique.

Selon une troisième caractéristique de l'invention, le moyen de mesure de la variation d'un paramètre résultant d'un effort physique consiste en une électrode bipolaire de mesure d'un signal électro-myographique d'au moins un muscle du périnée.

Selon une quatrième caractéristique de l'invention, le moyen de mesure de la variation d'un paramètre résultant d'un effort physique est un moyen de mesure de la variation de pression comportant au moins un capteur de pression, ce dernier étant apte à mesurer une variation, notamment une augmentation de la pression, au sein de la cavité vaginale ou rectale.

Selon une cinquième caractéristique de l'invention, la stimulation peut être faite directement sur le muscle assurant la continence urinaire ou indirectement par l'intermédiaire de ladite muqueuse.

Selon une sixième caractéristique de l'invention, le moyen de mesure consiste en un capteur de pression, apte à mesurer une variation de pression au sein de ladite cavité.

Selon une septième caractéristique de l'invention, le moyen de mesure consiste en une électrode de mesure d'un signal électromyographique des muscles du périnée.

Selon une huitième caractéristique de l'invention, le moyen de stimulation musculaire électrique consiste en au moins une électrode.

Selon une neuvième caractéristique de l'invention, le dispositif de sonde est constitué d'une première partie constituée par un tube cylindrique comportant, à l'une de ses extrémités, un moyen de préhension manuel dudit dispositif de sonde et, à l'extrémité opposée, une deuxième partie sphérique, une première électrode de stimulation étant positionnée sur la périphérie du tube cylindrique, alors qu'une seconde électrode de stimulation est positionnée au niveau de la périphérie sur la partie sphérique.

Selon une dixième caractéristique de l'invention, le moyen de mesure de la pression comporte un ballonnet qui est placé au contact de la muqueuse vaginale ou de la muqueuse anale afin de détecter les variations de pression dans la cavité abdominale.

Plus spécifiquement, le ballonnet est associé à au moins un capteur de pression situé à l'intérieur du ballonnet et/ou à au moins une lamelle de contact en matériau déformable, positionnée à la surface dudit ballonnet.

La présente invention comporte de nombreux avantages. D'une part, il peut être porté en permanence par la personne afin d'éviter la perte d'urines, et ce en toute discrétion, sans aucun risque de fuites ou de mauvaises odeurs. De ce fait, le dispositif selon l'invention est particulièrement hygiénique. Par conséquent, le dispositif de sonde de l'invention permet de se substituer au port de couches, et d'éviter une opération chirurgicale ou des séances contraignantes de rééducation périnéale, aux résultats souvent aléatoires. Enfin, le présent dispositif peut aussi bien être porté par un homme que par une femme.

D'autres caractéristiques et avantages de l'invention ressortiront de la description détaillée qui va suivre des modes de réalisation non limitatifs de l'invention, en référence à l'unique figure annexée représentant, de manière schématique, un dispositif de sonde selon l'invention.

L'invention est définie par les revendications.

Les modes décrits ci-après qui ne sont pas couverts par les revendications ne font pas partie de l'invention.

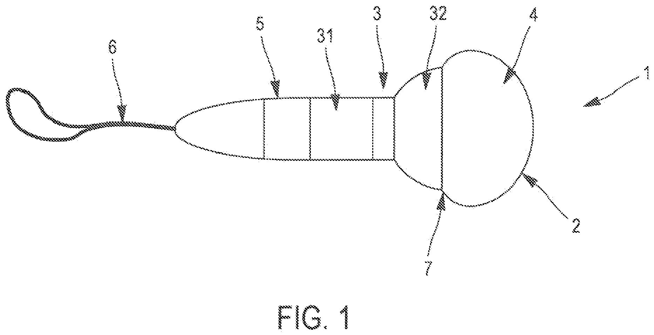

Tel que représentée sur la figure 1, la présente invention concerne un dispositif de sonde 1 qui, après introduction dans la cavité vaginale ou dans la cavité rectale d'un patient souffrant d'incontinence urinaire à l'effort, permet de gérer cette incontinence.

De manière particulièrement avantageuse, ce dispositif de sonde 1 selon l'invention est autonome. Autrement dit, ledit dispositif 1 comporte au moins un moyen d'alimentation autonome en énergie électrique, par exemple une batterie ou une pile bouton.

Ce moyen d'alimentation permet de faire fonctionner les différents composants dudit dispositif de sonde 1 de l'invention.

En effet, de manière préférentielle, ledit dispositif 1 comportet d'une part, au moins un moyen de mesure 2 de la variation d'un paramètre qui résulte d'un effort physique effectué par la personne portant ledit dispositif 1 et, d'autre part, au moins un moyen de stimulation musculaire électrique 3. Ce dernier est au contact d'au moins un muscle apte à maintenir la continence urinaire et permet de stimuler ce muscle lorsqu'un effort physique est détecté.

En particulier, le moyen de stimulation 3 est au contact direct ou à travers les muqueuse vaginale ou rectale, dudit au moins un muscle.

Le dispositif de sonde 1 selon l'invention est particulièrement intéressant car il permet de gérer l'incontinence urinaire chez une personne, homme ou femme, dès lors qu'une situation d'effort est détectée par ledit dispositif 1.

L'incontinence urinaire d'effort résulte notamment d'un affaiblissement des muscles du périnée (ou muscles du plancher pelvien, ou muscles pelviens) et/ou du muscle assurant la fermeture de la vessie, le sphincter urétral.

En effet, ces muscles, lorsqu'ils fonctionnent normalement, permettent de supporter une augmentation de la pression abdominale, due à une situation d'effort, comme un accès de toux, de rire, d'éternuements, ou un exercice physique. Plus précisément, une contraction des muscles périnéaux, lors d'un effort, renforce la pression de clôture urétrale assurée également par le sphincter et aboutit notamment à un maintien en position fermée de l'urètre.

En cas de diminution du tonus des muscles périnéaux, le sphincter urinaire ne peut assurer à lui seul une pression de clôture nécessaire à la continence. Par conséquent, lorsque la pression augmente dans la cavité abdominale suite à un effort, cette pression se répercute sur la vessie et les fuites d'urine peuvent survenir.

Les personnes touchées par l'incontinence urinaire sont principalement des femmes, peu importe leur âge. Dans certains cas, un traumatisme obstétrical peut être la cause d'un manque de tonicité du sphincter urinaire ainsi que d'un relâchement des muscles du plancher pelvien.

Toutefois, les hommes aussi peuvent être victimes d'incontinence urinaire, en particulier lorsqu'ils ont subi une ablation totale ou partielle de la prostate, ou prostatectomie. En effet, au cours de l'opération, il est possible que le sphincter urétral et/ou son innervation soi(en)t atteint(s) de manière accidentelle, rendant ledit sphincter moins efficace.

De ce fait, le dispositif de sonde 1 selon l'invention est susceptible d'être utilisé aussi bien par les hommes que par les femmes.

Dans un premier mode de réalisation du dispositif de sonde 1 de l'invention, le moyen de mesure 2 de la variation d'un paramètre résultant d'un effort physique est un moyen de mesure de la variation de pression comportant au moins un capteur de pression, ce dernier étant apte à mesurer une variation, notamment une augmentation de la pression, au sein de la cavité vaginale ou rectale.

Cette augmentation de la pression dans la cavité vaginale ou rectale résulte d'une augmentation de la pression dans l'abdomen, elle-même causée par une situation d'effort.

La détection d'une augmentation de pression dans la cavité par ledit moyen de mesure 2 permet de déclencher le moyen de stimulation électrique 3 du dispositif 1 positionné en contact avec au moins un muscle assurant la continence urinaire, de préférence un muscle du périnée. La stimulation électrique dudit muscle entraine une contraction électro-induite de ce dernier, aboutissant à la continence urinaire.

En particulier, le moyen de stimulation est au contact direct ou à travers les muqueuse vaginale ou rectale, dudit au moins un muscle. Ainsi, la stimulation peut être faite directement sur le muscle assurant la continence urinaire ou indirectement par l'intermédiaire de ladite muqueuse.

Préférentiellement, le moyen de mesure de la pression comporte un ballonnet 4 qui est placé au contact de la muqueuse vaginale ou de la muqueuse anale afin de détecter les variations de pression dans la cavité abdominale. A ces fins, le ballonnet 4 est associé à au moins un capteur de pression situé à l'intérieur du ballonnet 4 et/ou à au moins une lamelle de contact en matériau déformable, positionnée à la surface dudit ballonnet 4.

Aussi, par application du dispositif de sonde 1 selon l'invention, les fuites urinaires sont évitées.

En particulier, le ballonnet 4 est placé à une extrémité de la sonde. Plus particulièrement, ladite extrémité est destinée à être introduite jusqu'au contact avec le col de l'utérus ou au-delà du canal anal.

Le mode de réalisation du dispositif 1 décrit précédemment permet d'éviter l'incontinence urinaire d'effort lorsque les muscles, notamment périnéaux, ne présentent plus aucune activité. Dans ce cas de figure, comme indiqué, le paramètre résultant d'un effort qui est mesuré est la variation de pression, comme cela a été décrit.

Toutefois, il est également envisageable que la personne atteinte d'incontinence urinaire d'effort présenté encore une faible contraction des muscles périnéaux, mais insuffisante pour éviter une perte de liquide urinaire.

Dans ce cas de figure, correspondant au second mode de réalisation du dispositif de sonde 1 selon l'invention, le moyen de mesure 2 de la variation d'un paramètre résultant d'un effort physique consiste en une électrode bipolaire de mesure d'un signal électro-myographique d'au moins un muscle du périnée, non représentée sur la figure jointe.

En d'autres termes, le paramètre mesuré ici consiste en un courant électrique accompagnant l'activité musculaires, faible mais existante, notamment du périnée, cette activité résultant d'un effort physique.

Dans ce mode de réalisation particulier, le moyen de stimulation, de préférence également une électrode bipolaire en contact des muscles périnéaux, va permettre, par une stimulation électro-induite, d'amplifier l'activité musculaire existante et provoquer ainsi une contraction suffisante du muscle afin de maintenir la continence urinaire.

Le dispositif de sonde 1 selon l'invention permet donc, de manière particulièrement intéressante, de gérer l'incontinence urinaire d'effort à la fois chez des patients capables de produire une faible contraction musculaire périnéale et chez des patients dont le périnée ne présente plus aucune activité.

Dans un mode de réalisation avantageux, le dispositif de sonde 1 de l'invention comporte à la fois un moyen de mesure de la variation de la pression et une électrode bipolaire de mesure d'un signal électro-myographique détectant une activité électrique des muscles périnéaux,

En outre, il est également envisageable de calibrer le niveau de pression ou de puissance de signal électro-myographique aboutissant au déclenchement du moyen de stimulation électrique 3, et ce dans le but d'adapter le dispositif 1 au niveau d'incontinence de la patiente ou du patient.

En ce qui concerne à présent ledit moyen de stimulation électrique 3 du dispositif de sonde 1, positionné au contact d'au moins un muscle apte à maintenir la continence, celui-ci comporte, avantageusement, deux électrodes de stimulation 31, 32.

Lors d'une augmentation de pression abdominale, le moyen de mesure de ladite pression active le moyen de stimulation 3 des muscles périnéaux, Le cas échéant, l'intensité et la pente d'établissement de cette stimulation sont proportionnelles à la vitesse d'établissement et au niveau de la pression.

Les électrodes 31 et 32 étant placées au niveau des plans musculaires profonds du périnée, la stimulation ainsi établie provoque via ces électrodes 31 et 32 la contraction dudit périnée et accroît la pression de clôture urétrale.

De plus, le capteur qui active la contraction électro-induite est réglable et permet d'activer ladite stimulation à un seuil inférieur au seuil de pression à partir duquel se produit la fuite.

Plus généralement, le dispositif comprend le ballonnet 4 à une extrémité, et au moins une électrode 32 avant le ballonnet 4, puis, de préférence, l'autre électrode 31 avant la première électrode 32, Les positions des électrodes 31 et 32 sont adaptées à la stimulation dudit au moins un muscle.

De manière préférentielle, illustrée sur la figure 1 jointe, le dispositif de sonde 1 selon l'invention est constitué d'une première partis constituée par un tube cylindrique 5 comportant, à l'une de ses extrémités, un moyen de préhension manuel 6 dudit dispositif 1 et, à l'extrémité opposée, une deuxième partie 7 sensiblement sphérique.

Cette partie sphérique 7 comprend, avantageusement, le ballonnet 4 avec, à sa surface, au moins un capteur de pression et/ou à au moins une lamelle de contact en matériau déformable. De préférence, le ballonnet 4 est gonflable par un moyen approprié pouvant constituer le moyen de préhension 6. Le moyen de gonflage peut être une pompe. De préférence, le capteur de pression est compensé en température.

Une première électrode de stimulation 31 peut être positionnée sur la périphérie du tube cylindrique 5.

Pour ce qui est de la seconde électrode de stimulation 32, celle-ci est préférentiellement positionnée au niveau de la périphérie de la partie sphérique 7 du dispositif de sonde 1 selon l'invention.

Le dispositif de sonde 1 selon l'invention permet de gérer et d'empêcher l'incontinence urinaire de manièxe permanente, tout en évitant les inconvénients liés aux solutions proposées actuellement dans l'état de la technique.

Par conséquent, l'utilisateur du présent dispositif 1 sera totalement autonome, et ne sera plus sujet aux fuites urinaires.

En effet, au moment opportun, le dispositif 1 entrainera une stimulation électrique des muscles du périnée, engendrant une contraction électro-induite de ce dernier. On comprend ainsi que, au moyen du présent dispositif, on évite une stimulation intempestive des muscles périnéaux.

En outre, grâce à ce dispositif de sonde 1, au fur et à mesure des stimulations électriques des muscles, il est envisageable que le réflexe de verrouillage et le réflexe myotatique du périnée redevienent fonctionnels.

## Revendications

1. Dispositif de sonde (1)autonome et endocavitaire, pour la gestion de l'incontinence à l'effort, ladite sonde (1) étant apte introduite dans la cavité vaginale ou rectale d'une personne, le dispositif de sonde (1) comportant au moins un moyen d'alimentation autonome en énergie électrique qui alimente, d'une part, un moyen de mesure (2) de la variation d'un paramètre résultant d'un effort physique, et d'autre part, au moins un moye de stimulation musculaire électrique (3) au contact d'au moins un muscle apte à maintenir la continence urinaire, ledit moyen de stimulation électrique étant déclenché en cas de variation dudit paramètre, **caractérisé en ce que** le dispositif de sonde (1) étant configuré pour:
- mesurer, à l'aide du moyen de mesure (2), une augmentation de la pression au sein de la cavité vaginale ou rectale ; et
- activer le moyen de stimulation musculaire électrique (3) de sorte que l'intensité et la pente d'établissement de la stimulation sont proportionnelles à la vitesse d'établissement et au niveau de la pression mesurée par le moyen de mesure.

2. Dispositif de sonde (1) selon la revendication 1, **caractérisé en ce qu'**il est configuré pour activer la stimulation à un seuil inférieur au seuil de pression à partir duquel se produit la fuite.

3. Dispositif de sonde (1) selon l'une des revendications 1 et 2, **caractérisé en ce que** le paramètre mesuré consiste en un courant électrique accompagnant l'activité musculaire, faible mais existante, notamment du périnée, cette activité résultant d'un effort physique.

4. Dispositif de sonde (1) selon l'une des revendications 1 à 3, le moyen de mesure (2) de la variation d'un paramètre résultant d'un effort physique consiste en une électrode bipolaire de mesure d'un signal électro-myographique d'au moins un muscle du périnée.

5. Dispositif de sonde (1) selon l'une des revendications 1 et 2, **caractérisé en ce que** le moyen de mesure (2) de la variation d'un paramètre résultant d'un effort physique est un moyen de mesure de la variation de pression comportant au moins un capteur de pression, ce dernier étant apte à mesurer une variation, notamment une augmentation de la pression, au sein de la cavité vaginale ou rectale.

6. Dispositif de sonde (1) selon l'une des revendications 1 à 5, **caractérisé en ce que** le dispositif est configuré pour une stimulation faite directement sur le muscle assurant la continence urinaire ou indirectement par l'intermédiaire de ladite muqueuse.

7. Dispositif de sonde (1) selon l'une des revendications 1 à 6, **caractérisé par le fait que** ledit moyen de mesure (3) consiste en un capteur de pression, apte à mesurer une variation de pression au sein ladite cavité.

8. Dispositif de sonde (1) selon l'une des revendications 1 à 6, **caractérisé par le fait que** ledit moyen de mesure (3) consiste en une électrode de mesure d'un signal électro-myographique des muscles du périnée.

9. Dispositif de sonde (1) selon l'une des revendications 1 à 8, **caractérisé par le fait que** ledit moyen de stimulation musculaire électrique consiste en au moins une électrode.

10. Dispositif de sonde (1) selon la revendication 9, **caractérisé en ce qu'**il est constitué d'une première partie constituée par un tube cylindrique (5) comportant, à l'une de ses extrémités, un moyen de préhension manuel (6) dudit dispositif de sonde (1) et, à l'extrémité opposée, une deuxième partie sphérique (7), une première électrode de stimulation (31) étant positionnée sur la périphérie du tube cylindrique (5), alors qu'une seconde électrode de stimulation (32) est positionnée au niveau de la périphérie de la partie sphérique (7).

11. Dispositif de sonde (1) selon l'une des revendications 1 à 10, **caractérisé en ce que** le moyen de mesure (3) de la pression comporte un ballonnet (4) qui est placé au contact de la muqueuse vaginale ou de la muqueuse anale afin de détecter les variations de pression dans la cavité abdominale.

12. Dispositif de sonde (1) selon la revendication 11, **caractérisé en ce que** le ballonnet (4) est associé à au moins un capteur de pression situé à l'intérieur du ballonnet (4) et/ou à au moins une lamelle de contact en matériau déformable, positionnée à la surface dudit ballonnet (4).

## Patentansprüche

1. Autonome und endokavitäre Sondenvorrichtung (1) zur Behandlung von Stressinkontinenz, wobei die Sonde (1) in die Vaginal- oder Rektalhöhle einer Person einführbar ist, wobei die Sondenvorrichtung (1) mit mindestens einem Mittel zum autonomen Zuführen von elektrischer Energie, das zum einen einMessmittel (2) der Änderung eines Parameters, die aus einer körperlichen Anstrengung resultiert, und zum anderen mindestens ein Mittel zur elektrischen Muskelstimulation (3) umfasst, das in Kontakt mit mindestens einem Muskel ist, der in der Lage ist, die Urinkontinenz aufrechtzuerhalten, wobei das elektrische Stimulationsmittel bei Änderung des Parameters ausgelöst wird, **dadurch gekennzeichnet, dass** die Sondenvorrichtung (1) konfiguriert ist für:
- das Messen eines Druckanstiegs in der Vaginal- oder Rektalhöhle mit Hilfe der Messmittel (2); und
- das Aktivieren des Mittels zur elektrischen Muskelstimulation (3), so dass die Intensität und die Steigerung des Stimulationsaufbaus proportional zur Aufbaurate und zu der Höhe des von dem Messmittel gemessenen Drucks sind.

2. Sondenvorrichtung (1) nach Anspruch 1, **dadurch gekennzeichnet, dass** sie konfiguriert ist, um die Stimulation bei einer Schwelle unterhalb der Druckschwelle zu aktivieren, ab welcher der Urinabgang auftritt.

3. Sondenvorrichtung (1) nach einem der Ansprüche 1 und 2, **dadurch gekennzeichnet, dass** der gemessene Parameter aus einem elektrischen Strom besteht, der die Muskelaktivität, insbesondere des Perineums, die schwach aber vorhanden ist, begleitet, wobei diese Aktivität aus einer körperlichen Anstrengung resultiert.

4. Sondenvorrichtung (1) nach einem der Ansprüche 1 bis 3, wobei das Messmittel (2) der Änderung eines Parameters, die aus einer körperlichen Anstrengung resultiert, aus einer bipolaren Elektrode zum Messen eines elektromyographischen Signals von mindestens einem Perinealmuskel besteht.

5. Sondenvorrichtung (1) nach einem der Ansprüche 1 und 2, **dadurch gekennzeichnet, dass** das Messmittel (2) der Änderung eines Parameters, die aus einer körperlichen Anstrengung resultiert, ein Messmittel der Druckänderung mit mindestens einem Drucksensor ist, der eine Änderung, insbesondere einen Druckanstieg, innerhalb der Vaginal- oder Rektalhöhle messen kann.

6. Sondenvorrichtung (1) nach einem der Ansprüche 1 bis 5, **dadurch gekennzeichnet, dass** die Stimulation direkt auf den Muskel ausgeübt werden kann, der die Urinkontinenz bereitstellt, oder indirekt über die Schleimhaut.

7. Sondenvorrichtung (1) nach einem der Ansprüche 1 bis 6, **dadurch gekennzeichnet, dass** das Messmittel (3) aus einem Drucksensor besteht, der eine Druckänderung innerhalb des Hohlraums messen kann.

8. Sondenvorrichtung (1) nach einem der Ansprüche 1 bis 6, **dadurch gekennzeichnet, dass** das Messmittel (3) aus einer Elektrode zum Messen eines elektromyographischen Signals der Perinealmuskeln besteht.

9. Sondenvorrichtung (1) nach einem der Ansprüche 1 bis 8, **dadurch gekennzeichnet, dass** das Mittel zur elektrischen Muskelstimulation aus mindestens einer Elektrode besteht.

10. Sondenvorrichtung (1) nach Anspruch 9, **dadurch gekennzeichnet, dass** sie aus einem ersten Teil besteht, der aus einem zylindrischen Rohr (5) besteht, das an einem seiner Enden ein manuelles Greifmittel (6) der Sondenvorrichtung (1) aufweist, und am gegenüberliegenden Ende einen zweiten kugelförmigen Teil (7), wobei eine erste Stimulationselektrode (31) am Umfang des zylindrischen Rohrs (5) angeordnet ist, während eine zweite Stimulationselektrode (32) in Höhe des Umfangs des kugelförmigen Teils (7) angeordnet ist.

11. Sondenvorrichtung (1) nach einem der Ansprüche 1 bis 10, **dadurch gekennzeichnet, dass** das Druckmessmittel (3) einen Ballon (4) umfasst, der in Kontakt mit der Vaginalschleimhaut oder der Analschleimhaut gebracht wird, um Druckänderungen in der Bauchhöhle zu erfassen.

12. Sondenvorrichtung (1) nach Anspruch 11, **dadurch gekennzeichnet, dass** dem Ballon (4) mindestens ein im Ballon (4) befindlicher Drucksensor und/oder mindestens eine Kontaktleiste zugeordnet ist, die aus verformbarem Material besteht, das auf der Oberfläche des Ballons (4) angeordnet ist.

## Claims

1. Self-contained and endocavitary probe device (1) for managing stress incontinence, said probe (1) being insertable into the vaginal or rectal cavity of a person, the probe device (1) comprising at least one self-contained electrical power supply means which supplies power both to a means (2) for measuring the change in a parameter resulting from physical stress, and to at least one electrical muscle stimulation means (3) in contact with at least one muscle capable of maintaining urinary continence, said electrical stimulation means being triggered in case of a change in said parameter, **characterized in that** the probe device (1) is configured to:
- measure, using the measuring means (2), an increase in the pressure within the vaginal or rectal cavity; and
- activate the electrical muscle stimulation means (3) such that the intensity and slope of establishment of the stimulation are proportional to the rate of establishment and to the level of the pressure measured by the measuring means.

2. Probe device (1) according to claim 1, **characterized in that** the device is configured to activate the stimulation at a threshold below the pressure threshold at which the leak occurs.

3. Probe device (1) according to either claim 1 or claim 2, **characterized in that** the measured parameter consists of an electric current accompanying the weak but existing muscular activity, in particular from the perineum, said activity resulting from physical stress.

4. Probe device (1) according to any of claims 1 to 3, **characterized in that** the means (2) for measuring the change in a parameter resulting from physical stress consists of a bipolar electrode for measuring an electromyographic signal of at least one perineal muscle.

5. Probe device (1) according to either claim 1 or claim 2, **characterized in that** the means (2) for measuring the change in a parameter resulting from physical stress is a means for measuring the change in pressure comprising at least one pressure sensor, this being able to measure a change, in particular an increase, in pressure within the vaginal or rectal cavity.

6. Probe device (1) according to any of claims 1 to 5, **characterized in that** the stimulation can be carried out directly on the muscle responsible for urinary continence or indirectly via said mucosa.

7. Probe device (1) according to any of claims 1 to 6, **characterized in that** said measuring means (3) consists of a pressure sensor which is able to measure a change in pressure within said cavity.

8. Probe device (1) according to any of claims 1 to 6, **characterized in that** said measuring means (3) consists of an electrode for measuring an electromyographic signal of the perineal muscles.

9. Probe device (1) according to any of claims 1 to 8, **characterized in that** said electrical muscle stimulation means consists of at least one electrode.

10. Probe device (1) according to claim 9, **characterized in that** the device consists of a first portion composed of a cylindrical tube (5) comprising, at one of the ends thereof, a means (6) for manually gripping said probe device (1) and, at the opposite end, a second spherical portion (7), a first stimulation electrode (31) being positioned on the circumference of the cylindrical tube (5), while a second stimulation electrode (32) is positioned on the circumference of the spherical portion (7).

11. Probe device (1) according to any of claims 1 to 10, **characterized in that** the means (3) for measuring the pressure comprises a balloon (4) which is placed in contact with the vaginal mucosa or the anal mucosa in order to detect changes in pressure within the abdominal cavity.

12. Probe device (1) according to claim 11, **characterized in that** the balloon (4) is associated with at least one pressure sensor located inside the balloon (4) and/or with at least one contact strip made from deformable material and positioned on the surface of said balloon (4).
